# EUROPÄISCHE PATENTANMELDUNG

(11) **EP 2 487 251 A1**
(43) Veröffentlichungstag der Anmeldung: **15.08.2012**
(21) Anmeldenummer: 11154261.9
(22) Anmeldetag: 13.02.2011
(51) Int. Cl.: C12Q 1/68

(54) **Markersequenzen für die Diagnose von Prostatakarzinom und deren Verwendung**

(71) Anmelder: Protagen AG, 44227 Dortmund (DE)
(72) Erfinder: Lueking, Angelika, 44892 Bochum (DE); Kowald, Axel, 44892 Bochum (DE); Bartsch, Georg, 6072 Lans (AT); Klocker, Helmut, 6401 Inzing (AT)
(74) Vertreter: Simandi, Claus

(57) **Zusammenfassung**

Die vorliegende Erfindung betrifft neue Markersequenzen für Prostatakarzinom unter Ausschluss von Prostataentzündungserkrankungen oder Diabetes oder Polymorbidität und deren diagnostische Verwendung samt einem Verfahren zum Screenen von potentiellen Wirkstoffen für solche Prostata - Erkrankungen mittels dieser Markersequenzen. Ferner betrifft die Erfindung eine diagnostische Vorrichtung enthaltend solche Markersequenzen für Prostatakarzinom, insbesondere ein Proteinbiochip und dessen Verwendung.

## Beschreibung

Die vorliegende Erfindung betrifft neue Markersequenzen für Prostatakarzinom (syn.: Prostatakrebs) unter Ausschluss von Prostataentzündungserkrankungen oder Diabetes oder Polymorbidität und deren diagnostische Verwendung samt einem Verfahren zum Screenen von potentiellen Wirkstoffen für solche Prostata - Erkrankungen mittels dieser Markersequenzen. Ferner betrifft die Erfindung eine diagnostische Vorrichtung enthaltend solche Markersequenzen für Prostatakarzinom, insbesondere ein Proteinbiochip und dessen Verwendung.

Proteinbiochips gewinnen eine zunehmende industrielle Bedeutung in der Analytik und Diagnostik sowie in der Pharmaentwicklung. Proteinbiochips haben sich als Screeninginstrumente etabliert.

Hierbei wird die schnelle und hochparallele Detektion einer Vielzahl spezifisch bindender Analysemoleküle in einem einzigen Experiment ermöglicht. Zur Herstellung von Proteinbiochips ist es erforderlich, die benötigten Proteine zur Verfügung zu haben. Hierzu haben sich insbesondere Protein-Expressionsbibliotheken etabliert. Die Hochdurchsatz-Klonierung von definierten offenen Leserahmen ist eine Möglichkeit (Heyman, J.A., Cornthwaite, J., Foncerrada, L., Gilmore, J.R., Gontang, E., Hartman, K.J., Hernandez, C.L., Hood, R., Hull, H.M., Lee, W.Y., Marcil, R., Marsh, E.J., Mudd, K.M., Patino, M.J., Purcell, T.J., Rowland, J.J., Sindici, M.L. and Hoeffler, J.P. (1999) Genome-scale cloning and expression of individual open reading frames using topoisomerase I-mediated ligation. Genome Res, 9, 383-392; Kersten, B., Feilner, T., Kramer, A., Wehrmeyer, S., Possling, A., Witt, I., Zanor, M.I., Stracke, R., Lueking, A., Kreutzberger, J., Lehrach, H. and Cahill, D.J. (2003) Generation of Arabidopsis protein chip for antibody and serum screening. Plant Molecular Biology, 52, 999-1010; Reboul, J., Vaglio, P., Rual, J.F., Lamesch, P., Martinez, M., Armstrong, C.M., Li, S., Jacotot, L., Bertin, N., Janky, R., Moore, T., Hudson, J.R., Jr., Hartley, J.L., Brasch, M.A., Vandenhaute, J., Boulton, S., Endress, G.A., Jenna, S., Chevet, E., Papasotiropoulos, V., Tolias, P.P., Ptacek, J., Snyder, M., Huang, R., Chance, M.R., Lee, H., Doucette-Stamm, L., Hill, D.E. and Vidal, M. (2003) C. elegans ORFeome version 1.1: experimental verification of the genome annotation and resource for proteome-scale protein expression. Nat Genet, 34, 35-41.; Walhout, A.J., Temple, G.F., Brasch, M.A., Hartley, J.L., Lorson, M.A., van den Heuvel, S. and Vidal, M. (2000) GATEWAY recombinational cloning: application to the cloning of large numbers of open reading frames or ORFeomes. Methods Enzymol, 328, 575-592). Allerdings hängt ein solcher Ansatz stark mit dem Fortschritt der Genom-Sequenzierungsprojekte und der Annotierung dieser Gensequenzen zusammen. Darüber hinaus ist die Bestimmung der exprimierten Sequenz aufgrund differenzieller Spleißvorgänge nicht immer eindeutig. Dieses Problem kann durch die Anwendung von cDNA-Expressionsbibliotheken umgangen werden (Büssow, K., Cahill, D., Nietfeld, W., Bancroft, D., Scherzinger, E., Lehrach, H. and Walter, G. (1998) A method for global protein expression and antibody screening on high-density filters of an arrayed cDNA library. Nucleic Acids Research, 26, 5007-5008; Büssow, K., Nordhoff, E., Lübbert, C., Lehrach, H. and Walter, G. (2000) A human cDNA library for high-throughput protein expression screening. Genomics, 65, 1-8; Holz, C., Lueking, A., Bovekamp, L., Gutjahr, C., Bolotina, N., Lehrach, H. and Cahill, D.J. (2001) A human cDNA expression library in yeast enriched for open reading frames. Genome Res, 11, 1730-1735; Lueking, A., Holz, C., Gotthold, C., Lehrach, H. and Cahill, D. (2000) A system for dual protein expression in Pichia pastoris and Escherichia coli, Protein Expr. Purif., 20, 372-378). Hierbei wird die cDNA eines bestimmten Gewebes in einen bakteriellen oder einen eukaryotischen Expressionsvektor, wie z.B. Hefe, einkloniert. Die für die Expression verwendeten Vektoren zeichnen sich im Allgemeinen dadurch aus, dass sie induzierbare Promotoren tragen, mit denen sich der Zeitpunkt der Proteinexpression steuern lässt. Darüber hinaus weisen Expressionsvektoren Sequenzen für so genannte Affinitätsepitope oder -proteine auf, die zum einen den spezifischen Nachweis der rekombinanten Fusions-Proteine mittels eines gegen das Affinitätsepitop gerichteten Antikörpers erlauben, zum anderen wird die spezifische Aufreinigung über Affinitätschromatographie (IMAC) ermöglicht.

Beispielsweise wurden die Genprodukte einer cDNA-Expressionsbibliothek aus humanem fötalem Hirngewebe in dem bakteriellen Expressionssystem Escherichia coli im Hochdichte-Format auf einer Membran angeordnet und konnten erfolgreich mit unterschiedlichen Antikörpern gescreent werden. Es konnte gezeigt werden, dass der Anteil an Volllänge-Proteinen bei mindestens 66% liegt. Die rekombinanten Proteine aus Expressionsbibliotheken konnten darüber hinaus im Hochdurchsatz exprimiert und aufgereinigt werden (Braun P., Hu, Y., Shen, B., Halleck, A., Koundinya, M., Harlow, E. and LaBaer, J. (2002) Proteome-scale purification of human proteins from bacteria. Proc Natl Acad Sci U S A, 99, 2654-2659; Büssow (2000) supra; Lueking, A., Horn, M., Eickhoff, H., Büssow, K., Lehrach, H. and Walter, G. (1999) Protein microarrays for gene expression and antibody screening. Analytical Biochemistry, 270, 103-111). Solche Proteinbiochips auf der Basis von cDNA-Expressionsbibliotheken sind insbesondere Gegenstand der WO 99/57311 und WO 99/57312.

Ferner sind neben Antigen-präsentierenden Proteinbiochips ebenfalls Antikörper-präsentierende Anordnungen beschrieben (Lal et al (2002) Antibody arrays: An embryonic but rapidly growing technology, DDT, 7, 143-149; Kusnezow et al. (2003), Antibody microarrays: An evaluation of production parameters, Proteomics, 3, 254-264).

Es besteht jedoch ein hohes Bedürfnis indikationsspezifische diagnostische Vorrichtungen, wie einen Proteinbiochip, bereitzustellen.

Zu den Laborparametern gehören die saure Phosphatase (SP) und das prostataspezifische Antigen (PSA) zur Diagnose des Prostatakarzinom. Vor allem das PSA hat derzeit einen hohen Stellenwert in der Diagnostik. Es ist spezifisch für die Prostata, allerdings nicht für ein Tumorleiden, sondern kann auch bei Entzündungen, benigner Prostatahyperplasie, einem Harnverhalt oder ohne ersichtlichen Grund erhöht sein. Ein Wert über 4 ng/ml gilt bereits als abklärungsbedürftig. WO2010/000874 der Anmelderin beschreibt bereits die Diagnose von Prostatakarzinom und Prostataentzündungen mittels einem Proteinbiochip und stellt bestimmte diagnostische Markersequenzen zur Verfügung.

Nachteilig ist jedoch, dass diese Marker ebenfalls für die Diagnose von Prostataentzündung geeignet sind. Ferner ist im Stand der Technik nachteilig, dass aufgrund anderen Indikationen, wie Prostataentzündungen oder Diabetes, Falsch-positive Markersequenzen identifiziert werden können.

Die Aufgabe der vorliegenden Erfindung ist jedoch die Bereitstellung von verbesserten Markersequenzen und deren diagnostische Verwendung zur Behandlung von Prostatakarzinom, wobei insbesondere eine Diskriminierung bzw. Ausschluss einer Prostataentzündung erfolgt.

Die Bereitstellung von spezifischen Markersequenzen erlaubt eine sichere Diagnose und Stratifizierung von Patienten mit Prostatakarzinom, insbesondere mittels einem Proteinbiochip.

Daher betrifft die Erfindung die Verwendung von Markersequenzen zur Diagnose von Prostatakarzinom, wobei mindestens eine Markersequenz einer cDNA ausgewählt aus der Gruppe SEQ 1 - 246 oder jeweils ein dafür kodierendes Protein oder jeweils einer Teilsequenz oder Fragment davon (nachstehend: erfindungsgemäße Markersequenzen) an oder von einem zu untersuchenden Patienten bestimmt wird.

Die erfindungsgemäßen Markersequenzen konnten mittels differentiellem Screenen von Proben und zwar gesunder Probanden mit Patientenproben mit Prostatakarzinom auf einem Proteinbiochip identifiziert werden. Zur Abgrenzung und Diskriminierung von Prostataentzündungserkrankungen oder Diabetes werden die erfindungsgemäßen Markersequenzen mit Patientenproben von Prostataentzündungserkrankungen (z.B. alle Formen der Prostata-Hyperplasie, Prostatitis) oder Diabetes auf einem Proteinbiochip gegengeprüft. Lediglich solche erfindungsgemäßen Markersequenzen bleiben als Markersequenzen für Prostatakarzinom berücksichtigt, die nicht zugleich ebenfalls als Markersequenzen für Prostataentzündungserkrankungen oder Diabetes identifiziert wurden.

Dieses Vorgehen erlaubt ebenfalls vorteilhaft den Ausschluss einer Komorbidität bzw. Polymorbidität, z.B. bei vorliegender Prostataentzündungserkrankungen oder Diabetes. Andererseits werden im Zuge dieses Vorgehens Falsch-positive Markersequenzen ausgeschlossen.

Hierbei konnte erstmals mittels Proteinbiochips (siehe Beispiele) diese erfindungsgemäßen Markersequenzen sensitiv identifiziert werden.

Der Begriff "Prostatakarzinom" umfasst ausschließlich die Indikation Prostatakarzimon bzw. Prostatakrebs unter Ausschluss einer Ko- bzw. Polymorbidität (Definition z.B. nach Pschyrembel, de Gruyter, 261. Auflage (2007), Berlin). Die ggfs. überlappenden Erkrankungen einer Prostataentzündung oder Diabetes sind erfindungsgemäß strikt ausgeschlossen.

Der Begriff "Prostataentzündungserkrankungen" umfasst sämtliche Formen einer Prostatitis bis hin zu chronischen Formen einer Prostataentzündung, einschließlich einer Prostata-Hyperplasie, insbesondere benignen Prostata-Hyperplasie.

Im Rahmen dieser Erfindung wird unter "Diabetes mellitus", insbesondere der Typ II Diabetes mellitus (Insulinresistenz) verstanden, eine chronische Stoffwechselerkrankung, wobei die Insulinproduktion in den β-Zellen der Langerhansschen Inseln in der Bauchspeicheldrüse gestört ist oder Insulin zwar vorhanden ist, an seinem Zielort, den Zellmembranen, aber nicht richtig wirken kann. Die Folge dieser gestörten Insulinproduktion bzw. -wirkung sind erhöhte Blutzuckerwerte (Hyperglykämie). Beim diabetischen Krankheitsverlauf unterscheidet man zwischen Prädiabetes, bei dem es erst in seinem Endstadium zu einer laborchemisch nachweisbaren "gestörten Glukosetoleranz" kommt, und dem eigentlichen manifesten Diabetes mellitus. Am Anfang der prädiabetischen Krankheitsphase steht die Insulinresistenz. Nahezu zeitgleich entwickeln sich bereits eine endotheliale Dysfunktion, Hyperlipoproteinämie und hypertensive Kreislaufdysregulation.

Daher betrifft die Erfindung solche Markersequenzen die eine Ko- oder Polymorbidität mit anderen Indikationen, wie Prostataentzündungen oder Diabetes (in allen Formen), ausschließt.

In einer weiteren Ausführungsform werden daher mindestens 2 bis 5 oder 10, vorzugsweise 30 bis 50 Markersequenzen oder 50 bis 100 oder mehr Markersequenzen an oder von einem zu untersuchenden Patienten bestimmt.

In einer weiteren Ausführungsform der Erfindung können die erfindungsgemäßen Markersequenzen ebenfalls mit bekannten Biomarkern für diese Indikation kombiniert, ergänzt oder erweitert werden. Besonders bevorzugt sind ebenfalls solche Marker, wie in WO2010/000874 offenbart.

In einer bevorzugten Ausführungsform erfolgt die Bestimmung der Markersequenzen außerhalb des menschlichen Körpers und die Bestimmung erfolgt in einer ex vivo / in vitro Diagnose.

In einer weiteren Ausführungsform der Erfindung betrifft die Erfindung die Verwendung von Markersequenzen als Diagnostika, wobei mindestens eine Markersequenz einer cDNA ausgewählt aus der Gruppe SEQ 1 - 246 oder jeweils ein dafür kodierendes Protein oder jeweils einer Teilsequenz oder Fragment davon ist.

Ferner betrifft die Erfindung ein Verfahren zur Diagnose von Prostatakarzinom, wobei a.) mindestens eine Markersequenz einer cDNA ausgewählt aus der Gruppe SEQ 1 - 246 oder jeweils ein dafür kodierendes Protein oder jeweils einer Teilsequenz oder Fragment davon auf einem festen Träger aufgebracht wird und b.) mit Körperflüssigkeit oder Gewebeauszug eines Patienten in Kontakt gebracht wird und c.) der Nachweis einer Wechselwirkung der Körperflüssigkeit oder Gewebeauszug mit den Markersequenzen aus a.) erfolgt.

Daher betrifft die Erfindung ebenfalls Diagnostika zur Diagnose von Prostatakarzinom jeweils ausgewählt aus der Gruppe SEQ 1 - 246 oder jeweils ein dafür kodierendes Protein oder jeweils einer Teilsequenz oder Fragment davon.

In einer besonders bevorzugten Ausführungsform sind die Markersequenzen SEQ 1 - 18 besonders bevorzugt und SEQ 19 - 56 bevorzugt, wobei wiederum niedere numerische Werte jeweils bevorzugt sind.

Der Nachweis einer solchen Wechselwirkung kann beispielsweise durch eine Sonde, insbesondere durch einen Antikörper erfolgen.

Daher betrifft die Erfindung ebenfalls die Aufgabe eine diagnostische Vorrichtung oder einen Assay, insbesondere einen Proteinbiochip, bereitzustellen, der für Prostatakarzinom eine Diagnose oder Untersuchung erlaubt.

Ferner betrifft die Erfindung ein Verfahren zum Stratifizieren, insbesondere zur Risikostratifizierung und / oder Therapiesteuerung eines Patienten mit einem Prostatakarzinom, wobei mindestens eine Markersequenz einer cDNA ausgewählt aus der Gruppe SEQ 1 - 246 oder jeweils ein dafür kodierendes Protein an einem zu untersuchenden Patienten bestimmt wird.

Ferner umfasst ist die Stratifizierung der Patienten mit Prostatakarzinom in neue oder etablierte Subgruppen des Prostatakarzinoms, sowie die sinnvolle Auswahl von Patientengruppen für die klinische Entwicklung von neuen Therapeutika. Der Begriff Therapiesteuerung umfasst ebenfalls die Einteilung von Patienten in Respondern und Nicht-Respondern bezüglich einer Therapie oder dessen Therapieverlauf.

"Diagnose" im Sinne dieser Erfindung bedeutet die positive Feststellung eines Prostatakarzinoms mittels der erfindungsgemäßen Markersequenzen sowie die Zuordnung der Patienten zu einem Prostatakarzinom. Der Begriff der Diagnose umfasst die medizinische Diagnostik und diesbezügliche Untersuchungen, insbesondere die in-vitro Diagnostik und Labordiagnostik, ebenfalls Proteomics und Nukleinsäureblots. Weitere Untersuchungen können zur Absicherung und zum Ausschluss anderer Krankheiten vonnöten sein. Daher umfasst der Begriff Diagnose ebenfalls die Differentialdiagnose von Prostatakarzinom mittels der erfindungsgemäßen Markersequenzen sowie die Prognose eines Prostatakarzinoms.

"Stratifizieren (auch: Stratifikation) oder Therapiesteuerung" im Sinne dieser Erfindung bedeutet, dass das erfindungsgemäße Verfahren Entscheidungen zur Behandlung und Therapie des Patienten erlaubt, sei es die Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf bzw. Ätiologie oder Klassifizierung einer Erkrankung, z.B. in einen neuen oder bestehenden Subtyp oder die Differenzierung von Krankheiten und dessen Patienten.

In einer weiteren Ausführungsform der Erfindung umfasst der Begriff "Stratifizierung" insbesondere die Risikostratifizierung mit der Prognose eines "outcome" eines nachteiligen gesundheitlichen Ereignisses.

Im Rahmen dieser Erfindung wird unter "Patient" ein beliebiger Proband - Mensch oder Säugetier - verstanden, mit der Maßgabe, dass der Proband auf Prostatakarzinom untersucht wird.

Der Begriff "Markersequenzen" im Sinne dieser Erfindung bedeutet, dass die cDNA oder das jeweils daraus erhältliche Polypeptid oder Protein signifikant für Prostatakarzinom sind. Beispielsweise können die cDNA oder das jeweils daraus erhältliche Polypeptid oder Protein eine Wechselwirkung mit Substanzen aus der Körperflüssigkeit oder Gewebeauszug eines Patienten mit Prostataentzündungserkrankungen bis hin zum Prostatakarzinom aufweisen (z.B. Antigen (Epitop) / Antikörper (Paratop) Wechselwirkung). Im Sinne der Erfindung bedeutet "wobei mindestens eine Markersequenz einer cDNA ausgewählt aus der Gruppe SEQ 1 - 246 oder jeweils ein dafür kodierendes Protein oder jeweils einer Teilsequenz oder Fragment davon an oder von einem zu untersuchenden Patienten bestimmt wird", dass eine Wechselwirkung zwischen der Körperflüssigkeit oder Gewebeauszuges eines Patienten und den erfindungsgemäßen Markersequenzen nachgewiesen wird. Eine solche Wechselwirkung ist z.B. eine Bindung, insbesondere eine bindende Substanz an mindestens einer erfindungsgemäßen Markersequenz oder im Fall einer cDNA die Hybridisierung mit einer geeigneten Substanz unter gewählten Bedingungen, insbesondere stringenten Bedingungen (z.B. wie üblich definiert in J. Sambrook, E.F. Fritsch, T. Maniatis (1989), Molecular cloning: A laboratory manual, 2nd Edition, Cold Spring Habor Laboratory Press, Cold Spring Habor, USA oder Ausubel, "Current Protocols in Molecular Biology", Green Publishing Associates and Wiley Interscience, N.Y. (1989)). Ein Beispiel für stringente Hybridisierungsbedingungen ist: Hybridisierung in 4 x SSC bei 65° C (alternativ in 50% Formamid und 4 X SSC bei 42° C), gefolgt von mehreren Waschschritten in 0,1 x SSC bei 65°C für insgesamt etwa eine Stunde. Ein Beispiel für wenig stringente Hybridisierungsbedingungen ist Hybridisierung in 4 x SSC bei 37° C, gefolgt von mehreren Waschritten in 1 x SSC bei Raumtemperatur.

Solche Substanzen sind erfindungsgemäß Bestandteil einer Körperflüssigkeit, insbesondere Blut, Vollblut, Blutplasma, Blutserum, Patientenserum, Urin, Cerebrospinalflüssigkeit, Synovialflüssigkeit oder eines Gewebeauszuges des Patienten.

In einer weiteren Ausführungsform der Erfindung können jedoch die erfindungsgemäßen Markersequenzen in einer signifikant höheren oder niedrigeren Expressionsrate oder Konzentration vorliegen, dass auf Prostatakarzinom hinweist. Hierbei wird mittels Proteomics oder Nukleinsäureblots die relativen Expressionsraten krank / gesund der erfindungsgemäßen Markersequenzen für Prostataentzündungserkrankungen, Prostatakarzinom bestimmt.

Die Markersequenzen verfügen in einer weiteren Ausführungsform der Erfindung über ein Erkennungssignal, welches an die zu bindende Substanz adressiert ist (z.B. Antikörper, Nukleinsäure). Erfindungsgemäß bevorzugt ist für ein Protein das Erkennungssignal ein Epitop und / oder Paratop und / oder Hapten und für eine cDNA eine Hybridisierungs- oder Bindungsregion.

Die erfindungsgemäßen Markersequenzen sind Gegenstand der Tabelle A und können durch den jeweilig zitierten Datenbankeintrag (auch mittels Internet:
http://www.ncbi.nlm.nih.gov/) eindeutig identifiziert werden (siehe in Tabelle A: dort Accession No.).

Daher betrifft die Erfindung ebenfalls die Volllängesequenzen der erfindungsgemäßen Marker und zwar wie in Tabelle 1 über den bekannten Datenbankeintrag gemäß Tabelle A definiert, nachstehend SEQ 247 - 452 genannt, siehe ebenfalls das zugehörige Sequenzprotokoll.

Weiterhin umfasst sind daher ebenfalls analoge Ausführungsformen von SEQ 247 - 452 zu den Markersequenzen SEQ 1-246, wie z.B. in den Ansprüchen dargelegt, da die erfindungsgemäßen SEQ 1-246 wiederum Teilsequenzen, zumindest mit hoher Homologie, darstellen. Die spezifischen Markersequenzen SEQ 1-246 sind jedoch erfindungsgemäß bevorzugt.

Weiterhin bevorzugt sind SEQ 247 - 260 und SEQ 261 - 294.

Erfindungsgemäß umfassen die Markersequenzen auch solche Modifikationen der cDNA-Sequenz und der entsprechenden Aminosäuresequenz, wie chemische Modifikation, wie Citrullinierung, Acetylierung, Phosphorylierung, Glykosilierung oder polyA-Strang und weiteren dem Fachmann einschlägig bekannte Modifikationen.

In einer weiteren Ausführungsform der Erfindung sind ebenfalls Teilsequenzen oder Fragmente der erfindungsgemäßen Markersequenzen umfasst. Insbesondere solche Teilsequenzen, die eine Identität von 95%, 90 %, insbesondere 80% oder 70 % mit den erfindungsgemäßen Markersequenzen aufweisen.

Teilsequenzen sind ebenfalls solche Sequenzen, die 50 bis 100 Nukleotide, 70-120 Nukleotide einer Sequenz der SEQ 1-452 aufweisen, oder davon erhältliche Peptide.

In einer weiteren Ausführungsform kann die jeweilige Markersequenz in unterschiedlichen Mengen in einen oder mehreren Bereichen auf einem festen Träger repräsentiert sein. Dies erlaubt eine Variation der Sensitivität. Die Bereiche können jeweils eine Gesamtheit von Markersequenzen aufweisen, d.h. eine genügende Zahl an verschiedenen Markersequenzen, insbesondere 2 bis 5 oder 10 oder mehr und ggfs. weiteren Nukleinsäuren und/oder Proteinen, insbesondere Biomarker. Bevorzugt sind jedoch mindestens 96 bis 25.000 (numerisch) oder mehr aus verschiedenen oder gleichen Markersequenzen und weiteren Nukleinsäuren und/oder Proteinen, insbesondere Biomarker. Weiterhin bevorzugt sind mehr als 2.500, besonders bevorzugt 10.000 oder mehr verschiedene oder gleiche Markersequenzen und ggfs. weiteren Nukleinsäuren und/oder Proteinen, insbesondere Biomarker.

Ein weiterer Gegenstand der Erfindung betrifft eine Anordnung von Markersequenzen enthaltend mindestens eine Markersequenz einer cDNA ausgewählt aus der Gruppe SEQ 1 - 452 oder jeweils ein dafür kodierendes Protein. Vorzugsweise enthält die Anordnung mindestens 2 bis 5 oder 10, vorzugsweise 30 bis 50 Markersequenzen oder 50 bis 100 oder mehr Markersequenzen.

Im Rahmen dieser Erfindung bedeutet "Anordnung" synonym "Array" und sofern dieser "Array" zur Identifizierung von Substanzen an Markersequenzen verwendet wird, ist hierunter ein "Assay" oder eine diagnostische Vorrichtung zu verstehen. In einer bevorzugten Ausführungsform ist die Anordnung derart gestaltet, dass die auf der Anordnung repräsentierten Markersequenzen in Form eines Gitters auf einem festen Träger vorliegen. Ferner sind solche Anordnungen bevorzugt, die eine hochdichte (high-density) Anordnung von Proteinbindern erlauben und die Markersequenzen gespottet werden. Solche hochdichte gespotteten Anordnungen sind beispielsweise in der WO 99/57311 und WO 99/57312 offenbart und können vorteilhaft in einem robotergestützten automatisierten High-Throughput Verfahren zur Anwendung kommen.

Im Rahmen dieser Erfindung umfasst jedoch der Begriff "Assay" oder diagnostische Vorrichtung ebenfalls solche Ausführungsformen einer Vorrichtung, wie ELISA, Bead-based Assay, Line Assay, Western Blot, immunchromatographische Verfahren (z.B. so genannte Lateral Flow Immunoassays) oder ähnliche immunologische Single- oder Multiplex-Nachweisverfahren. Ein Proteinbiochip im Sinne dieser Erfindung ist die systematische Anordnung von Proteinen auf einem festen Träger.

Die Markersequenzen der Anordnung sind auf einen festen Träger fixiert, vorzugsweise jedoch gespottet oder immobilisiert gar aufgedruckt, d.h. reproduzierbar aufgebracht. Ein oder mehrere Markersequenzen können mehrfach in der Gesamtheit aller Markersequenzen präsent sein und in unterschiedlichen Mengen bezogen auf einen Spot vorliegen. Ferner können die Markersequenzen auf dem festen Träger standardisiert sein (z.B. mittels serieller Verdünnungsreihen von z.B. Humanglobulinen als interne Kalibratoren zur Datennormalisierung und quantitativen Auswertung).

Daher betrifft die Erfindung einen Assay oder Proteinbiochip bestehend aus einer Anordnung enthaltend erfindungsgemäße Markersequenzen.

In einer weiteren Ausführungsform liegen die Markersequenzen als Clone vor. Solche Clone können beispielsweise mittels einer erfindungsgemäßen cDNA-Expressionsbibliothek erhalten werden (Büssow et al. 1998 (supra)). In einer bevorzugten Ausführungsform werden solche Expressionsbibliotheken enthaltend Clone mittels Expressionsvektoren aus einer exprimierenden cDNA Bibliothek bestehend aus den cDNA Markersequenzen erhalten. Diese Expressionsvektoren enthalten vorzugsweise induzierbare Promotoren. Die Induktion der Expression kann z.B. mittels eines Induktors, solche wie IPTG, erfolgen. Geeignete Expressionsvektoren sind beschrieben in Terpe et al. (Terpe T Appl Microbiol Biotechnol. 2003 Jan; 60(5):523-33).

Expressionsbibliotheken sind dem Fachmann bekannt, diese können nach Standardwerken, wie Sambrook et al, "Molecular Cloning, A laboratory handbook, 2nd edition (1989), CSH press, Cold Spring Harbor, New York hergestellt werden. Weiterhin bevorzugt sind solche Expressionsbibliotheken, die gewebespezifisch sind (z.B. humanes Gewebe, insbesondere humane Organe). Ferner sind erfindungsgemäß ebenfalls solche Expressionsbibliotheken mit eingeschlossen, die mittels exontrapping erhalten werden können. Statt Expressionsbibliothek kann synonym von einer Expressionsbank gesprochen werden. Weiterhin bevorzugt sind Proteinbiochips oder entsprechende Expressionsbibliotheken, die keine Redundanz aufweisen (so genannte: Uniclone®-Bibliothek) und nach den Lehren der WO 99/57311 und WO 99/57312 beispielsweise hergestellt werden können. Diese bevorzugten Uniclone- Bibliotheken weisen einen hohen Anteil an nicht-fehlerhaften vollständig exprimierten Proteinen einer cDNA-Expressionsbibliothek auf.

Im Rahmen dieser Erfindung können die Clone ebenfalls nicht abschließend solche sein, wie transformierte Bakterien, rekombinante Phagen oder transformierte Zellen von Säugern, Insekten, Pilzen, Hefen oder Pflanzen.

Die Clone werden auf einen festen Träger fixiert, gespottet oder immobilisiert.

Daher betrifft die Erfindung eine Anordnung, wobei die Markersequenzen als Clone vorliegen.

Zusätzlich können die Markersequenzen in der jeweiligen Form in Form eines Fusionsproteins vorliegen, welches beispielsweise mindestens ein Affinitätsepiptop oder "Tag" enthält. Der Tag kann ein solcher sein wie wie c-myc, His-Tag, Arg-tag, FLAG, alkalische Phosphatase, V5-Tag, T7-Tag oder Strep-Tag, HAT-tag, NusA, S-tag, SBP-tag, Thioredoxin, DsbA, ein Fusionsprotein, vorzugsweise eine Cellulose-bindende Domäne, grünfluoreszierendes Protein, Maltose bindendes Protein, calmodulin-bindendes Protein, Glutathione S-transferase oder lacZ enthalten.

In sämtlichen Ausführungsformen umfasst der Begriff "fester Träger" Ausführungen wie einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix. Ein Filter ist jedoch erfindungsgemäß bevorzugt.

Als Filter ist weiterhin PVDF, Nitrocellulose oder Nylon bevorzugt (z.B. Immobilon P Millipore, Protran Whatman, Hybond N+ Amersham).

In einer weiteren bevorzugten Ausführungsform der erfindungsgemäßen Anordnung entspricht diese einem Gitter, dass die Größenordnung einer Mikrotiterplatte (8-12 Wells Streifen, 96 Wells, 384 Wells oder mehr), eines Silizium-Wafers, eines Chips, eines massenspektrometrischen Targets oder einer Matrix besitzt.

In einer weiteren Ausführungsform betrifft die Erfindung einen Assay oder Proteinbiochip zum Identifizieren und Charakterisieren einer Substanz für Prostatakarzinom, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Ferner betrifft die Erfindung ein Verfahren zum Identifizieren und Charakterisieren einer Substanz für Prostatakarzinom, dadurch gekennzeichnet, dass eine erfindungsgemäße Anordnung oder Assay mit a.) mindestens einer zu untersuchenden Substanz in Kontakt gebracht wird und b.) ein Bindungserfolg nachgewiesen wird.

Die zu untersuchende Substanz kann ein beliebiges natives oder nicht-natives Biomolekül, ein synthetisches chemisches Molekül, eine Mischung oder eine Substanzbibliothek sein.

Nachdem die zu untersuchende Substanz eine Markersequenz kontaktiert, erfolgt die Auswertung des Bindungserfolges, die beispielsweise unter Verwendung mit handelsüblicher Image-Analyse Software (GenePix Pro (Axon Laboratories), Aida (Raytest), ScanArray (Packard Bioscience) erfolgt.

Die Visualisierung erfindungsgemäßer Protein-Protein-Wechselwirkungen (z.B. Protein an Markersequenz, wie Antigen/Antikörper) oder entsprechende "Mittel zum Nachweis des Bindungserfolges" kann beispielsweise mittels Fluoresenzmarkierung, Biotiniylierung, Radio-Isotopen-Markierung oder kolloidale Gold- oder Latex-Partikel-Markierung in üblicher Weise erfolgen. Ein Nachweis von gebundenen Antikörpern erfolgt mit Hilfe von sekundären Antikörpern, die mit handelsüblichen Reportermolekülen markiert sind (z.B. Cy-, Alexa-, Dyomics, FITC- oder ähnliche Fluoreszenzfarbstoffe, , kolloidale Gold- oder Latex-Partikel), oder mit Reporter-Enzymen wie alkalischer Phosphatase, Meerrettichperoxidase, usw. und den entsprechenden colorimetrischen, fluoreszenten oder chemolumineszenten Substraten. Eine Auslesung erfolgt z.B. mittels eines Microarray-Laserscanners, einer CCD-Kamera oder visuell.

In einer weiteren Ausführungsform betrifft die Erfindung ein Arzneimittel / Wirkstoff oder Prodrug für Prostatakarzinom entwickelt und erhältlich durch den Einsatz des erfindungsgemäßen Assays oder Proteinbiochip.

Daher betrifft die Erfindung ebenfalls die Verwendung einer erfindungsgemäßen Anordnung oder einem Assay zum Screenen von Wirkstoffen für Prostatakarzinom.

Daher betrifft die Erfindung in einer weiteren Ausführungsform ebenfalls ein Target zur Behandlung und Therapie von Prostatakarzinom, jeweils ausgewählt aus der Gruppe SEQ 1 - 246 oder jeweils ein dafür kodierendes Protein.

In einer weiteren Ausführungsform betrifft die Erfindung ebenfalls die Verwendung der erfindungsgemäßen Markersequenzen, vorzugsweise in Form einer Anordnung, als Affinitätsmaterial zur Durchführung einer Apherese bzw. iwS. einer Blutwäsche, wobei Substanzen aus Körperflüssigkeiten eines Patienten mit Prostatakarzinom, wie Blut oder Plasma, an die erfindungsgemäßen Markersequenzen binden und folglich der Körperflüssigkeit selektiv entzogen werden können. Entsprechende Vorrichtungen sind einschlägig bekannt, wie z.B. chromatographische Vorrichtungen enthaltend Beads, Kugeln oder chromatographisches Material, z.B. in einer Säule, die die erfindungsgemäßen Markersequenzen aufweisen und daher z.B. (Auto)Antikörper selektiv entziehen können.

Beispiele und Figuren:
Zehn oder mehr Patientenproben wurden individuell gegen eine cDNA Expressionsbibliothek gescreent. Die Prostatakarzinom - spezifischen Expressionsklone wurden ermittelt durch einen Vergleich mit zehn oder mehr gesunden Proben. Ferner wurden die identifizierten Marker gegen Proben von Prostataentzündungs- oder Diabetespatienten gegengeprüft. Falsch-positive Marker wurden entfernt. Die anschließende Identität der verbleibenden Markersequenzen wurde durch DNA-Sequenzierung ermittelt.

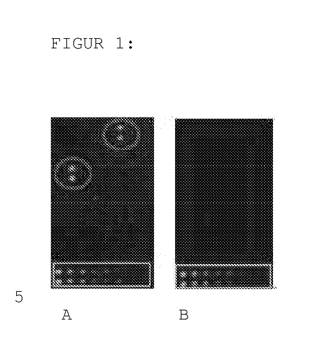

In Figur 1 wird das differentielle Screenen zwischen zwei Proteinbiochips aus jeweils einer cDNA-Expressionsbank eines Patienten und einem gesunden Probanden gezeigt. Die differentiellen Clone werden mittels Fluoresenzmarkierung nachgewiesen und bioinformatorisch ausgewertet.

Im Rahmen der Biomarkeridentifizierung werden verschiedene bioinformatische Analysen durchgeführt. Für jedes Serum werden mittels Microarray Reaktivitäten gegen ca. 2000 unterschiedliche Antigene gemessen. Diese Daten werden für ein Ranking der gespotteten Antigene bzgl. ihrer Differenzierungsfähigkeit zwischen gesunden und erkrankten Seren benutzt. Diese Auswertung wird mittels des nicht parametrischen Mann-Whitney Tests auf normalisierten Intensitätsdaten durchgeführt. Zur Normalisierung wird ein interner Standard benutzt, der auf jedem Chip mitgespottet wird. Da für jedes Antigen ein p-Wert berechnet wird, werden Methoden zur Korrektur des multiples Testens eingesetzt. Als sehr konservativer Ansatz wird eine Bonferroni Korrektur durchgeführt und zusätzlich wird die weniger restriktive False Discovery Rate (FDR) nach Benjamini & Hochberg berechnet. Desweiteren werden die Daten zur Klassifikation der Seren benutzt. Hierbei kommen unterschiedliche multivariate Methoden zum Einsatz. Dies sind Methoden aus den statistischen Lernverfahren wie Support Vector Machines (SVM), Neuronale Netze oder Klassifikationsbäume, sowie eine Schwellenwertmethode, welche sowohl zur Klassifikation als auch zur visuellen Repräsentation der Daten geeignet ist.

Zur Vermeidung von Overfitting wird eine 10fache Cross-Validierung der Daten durchgeführt.

**Tabelle A:**

| SEQ ID | gi Accession | Name | DNA Accession |
|---|---|---|---|
| 247 | gi\|15902723 | Neuro-oncological ventral antigen 2 (NOVA2) | NM_002516 |
| 248 | gi\|113411825 | Similar to Cyclin-L2 (Paneth cell-enhanced expression protein) transcript variant 1 (LOC727877) | NM_030937 |
| 249 | gi\|31543652 | Signal recognition particle 14kDa (homologous Alu RNA binding protein) (SRP14) | NM_003134 |
| 250 | gi\|42544158 | Heat shock 105kDa/110kDa protein 1 (HSPH1) | NM_006644 |
| 251 | gi\|113428756 | Zinc finger protein 154 (pHZ-92) (ZNF154) | NM_001085384 |
| 252 | gi\|32490571 | Erythrocyte membrane protein band 4.1-like 3 (EPB41L3) | NM_012307 |
| 253 | gi\|77628146 | Endoplasmic reticulum protein 29 (ERP29) transcript variant 1 | NM_006817 |
| 254 | gi\|22749232 | Zinc finger protein 579 (ZNF579) | NM_152600 |
| 255 | gi\|194097404 | Peptide chain release factor 3 | NM_018094 |
| 256 | gi\|217330633 | Serine/Threonine kinase 36 | NM_015690 |
| 257 | gi\|16507207 | Capicua homolog (Drosophila) (CIC) | NM_015125 |
| 258 | gi\|32261293 | Protein kinase interferon-inducible double stranded RNA dependent activator (PRKRA) | NM_003690 |
| 259 | gi\|49574506 | Neugrin neurite outgrowth associated (NGRN) transcript variant 1 | NM_001033088 |
| 260 | gi\|89031690 | Chromosome 10 genomic contig, reference assembly | NM_005876 |
| 261 | gi\|113427652 | Alveolar soft part sarcoma chromosome region candidate 1 (ASPSCR1) | NM_024083 |
| 262 | gi\|40353201 | OTU domain containing 5 (OTUD5) | NM_017602 |
| 263 | gi\|66932901 | SREBP cleavage-activating protein (SCAP) | NM_012235 |
| 264 | gi\|33624820 | Septin 6 (SEPT6) transcript variant V | NM_145800 |
| 265 | gi\|40807365 | Dihydrouridine synthase 1-linke (S. cerevisiae) (DUS1L) | NM_022156 |
| 266 | gi\|52851419 | Chromosome 6 open reading frame 153 (C6orf153) | NM_033112 |
| 267 | gi\|12597652 | 5'-nucleotidase domain containing 2 (NTSDC2) | NM_001134231 |
| 268 | gi\|113428394 | Plasticity-related gene 2 (PRG2) | XM_001129992 |
| 269 | gi\|40353728 | Ras and Rab interactor 3 (RIN3) | NM_024832 |
| 270 | gi\|55925649 | Transcription elongation factor A (SII)-like 2 (TCEAL2) | NM_080390 |
| 271 | gi\|34147459 | Coiled-coil domain containing 102A (CCDC102A) | NM_033212 |
| 272 | gi\|63003893 | Hypothetical protein LOC154467 Chromosome 6 open reading frame 129 (C6orf129) | NM_138493 |
| 273 | gi\|22538469 | Eomesodermin homolog (Xenopus laevis) (EOMES) | NM_005442 |
| 274 | gi\|34222261 | Tubulin, beta (TUBB) | NM_000972 |
| 275 | gi\|56676308 | Peptidylprolyl cis/trans isomerase NIMA-interacting 1 (PIN1) | NM_006221 |
| 276 | gi\|7669552 | Valosin-containing protein (VCP) | NM_007126 |
| 277 | gi\|34335231 | Creatine kinase brain (CKB) | NM_001823 |
| 278 | gi\|62414288 | Vimentin (VIM) | NM_003380 |
| 279 | gi\|71772259 | Ribosomal protein L5 (RPL5) | NM_000969 |
| 280 | gi\|223718111 | Solute carrier family 25 (mitochondrial carrier; phosphate carrier), member 3 (SLC25A3), nuclear gene encoding mitochondrial protein, transcript variant 3 | NM_213611 |
| 281 | gi\|33636763 | Ankyrin repeat domain 13B (ANKRD13B) | NM_152345 |
| 282 | gi\|38372936 | Chromatin modifying protein 2A (CHMP2A) transcript variant 1 | NM_014453 |
| 283 | gi\|134152707 | Arginine and glutamate rich 1 | NM_018011 |
| 284 | gi\|37577133 | Ubiquitin-conjugating enzyme E2M (UBC12 homolog yeast) (UBE2M) | NM_003968 |
| 285 | gi\|55925607 | Kelch-like 21 (Drosophila) (KLHL21) | NM_014851 |
| 286 | gi\|92859637 | Synaptotagmin V (SYT5) | NM_003180 |
| 287 | gi\|83776595 | CaM kinase-like vesicle-associated (CAMKV) | NM_024046 |
| 288 | gi\|14042969 | Integrin alpha FG-GAP repeat containing 3 (ITFG3) | NM_032039 |
| 289 | gi\|23111001 | V-maf musculoaponeurotic fibrosarcoma oncogene homolog F (avian) (MAFF) transcript variant 1 | NM_012323 |
| 290 | gi\|39725935 | Syntaxin 18 (STX18) | NM_016930 |
| 291 | gi\|194018519 | G1 to S phase transition 1 isoform 1 | NM_002094 |
| 292 | gi\|34147576 | RaP2 interacting protein 8 (RPIP8) | NM_001144825 |
| 293 | gi\|12597634 | B-cell CLL/lymphoma 11B (zinc finger protein) (BCL11B) transcript variant 2 | NM_022898 |
| 294 | gi\|22212941 | Ubiquitin associated protein 1 (UBAP1) | NM_016525 |
| 295 | gi\|38016910 | Stomatin (STOM) transcript variant 1 | NM_004099 |
| 296 | gi\|66879658 | ADP-ribosylation factor 1 (ARF1) transcript variant 4 | NM_001658 |
| 297 | gi\|68161512 | Cyclic AMP-regulated phosphoprotein 21 kD (ARPP-21) transcript variant 1 | NM_016300 |
| 298 | gi\|56181386 | STIP1 homology and U-box containing protein 1 (STUB1) | NM_005861 |
| 299 | gi\|49640010 | Tetratricopeptide repeat domain 3 (TTC3), transcript variant 2 | NM_001001894 |
| 300 | gi\|34222326 | HMP19 protein (HMP19) | NM_015980 |
| 301 | gi\|16445394 | Cadherin 18, type 2 (CDH18) | NM_004934 |
| 302 | gi\|5730103 | Thioredoxin-like 2 (TXNL2) | NM_006541 |
| 303 | gi\|113428396 | SHC (Src homology 2 domain containing) transforming protein 2 (SHC2) | XM_939572 |
| 304 | gi\|23111046 | Sorting nexin 5 (SNX5), transcript variant 1 | NM_152227 |
| 305 | gi\|13654275 | Queuine tRNA-ribosyltransferase 1 (tRNA-guanine transglycosylase) (QTRT1) | NM_031209 |
| 306 | gi\|82524843 | Multiple EGF-like-domains 6 (MEGF6) | NM_001409 |
| 307 | gi\|12232414 | Family with sequence similarity 59, member A (FAM59A) | NM_022751 |
| 308 | gi\|33286445 | Opioid growth factor receptor (OGFR) | NM_007346 |
| 309 | gi\|74048536 | Praja 1 (PJA1), transcript variant 2 | NM_001032396 |
| 310 | gi\|31652250 | Chromosome 3 open reading frame 19 (C3orf19) | NM_016474 |
| 311 | gi\|35250828 | Coatomer protein complex, subunit gamma (COPG) | NM_016128 |
| 312 | gi\|22748978 | Transmembrane protein 199 (TMEM199) | NM_152464 |
| 313 | gi\|68448524 | CD74 molecule, major histocompatibility complex, class II invariant chain (CD74), transcript variant 2 | NM_004355 |
| 314 | gi\|89111136 | Myosin, light chain 6B, alkali, smooth muscle and non-muscle (MYL6B) | NM_002475 |
| 315 | gi\|38679895 | Pleckstrin homology domain interacting protein (PHIP) | NM_017934 |
| 316 | gi\|17981697 | Cyclin-dependent kinase inhibitor 2C (p18, inhibits CDK4) (CDKN2C), transcript variant 1 | NM_001262 |
| 317 | gi\|149158722 | N-terminal EF-hand calcium binding protein 3 isoform 2 | NM_031232 |
| 318 | gi\|40807453 | Zinc finger protein 133 (ZNF133) | NM_003434 |
| 319 | gi\|7105395 | Ribosomal protein L29 (RPL29) | NM_000992 |
| 320 | gi\|111034854 | TMEM9 domain family, member B (TMEM9B) | NM_020644 |
| 321 | gi\|2749406 | Polymerase (RNA) I polypeptide D, 16kDa (POLR1D), transcript variant 2 | NM_152705 |
| 322 | gi\|61742795 | Basal cell adhesion molecule (Lutheran blood group) (BCAM), transcript variant 1 | NM_005581 |
| 323 | gi\|113425940 | Similar to eukaryotic translation initiation factor 3, subunit 8, 110kDa, transcript variant 4 (LOC728689) | XM_001132509 |
| 324 | gi\|34304357 | Nuclear factor of kappa light polypeptide gene enhancer in B-cells inhibitor-like 2 (NFKBIL2) | NM_013432 |
| 325 | gi\|4758985 | RAB11B, member RAS oncogene family (RAB11B) | NM_004218 |
| 326 | gi\|21361279 | Ts translation elongation factor, mitochondrial (TSFM) | NM_005726 |
| 327 | gi\|4503816 | Follicular lymphoma variant translocation 1 (FVT1) | NM_002035 |
| 328 | gi\|4110406 | Heterogeneous nuclear ribonucleoprotein D-like (HNRPDL), transcript variant 2 | NM_031372 |
| 329 | gi\|50345985 | ATP synthase, H+ transporting, mitochondrial F1 complex, beta polypeptide (ATP5B), nuclear gene encoding mitochondrial protein | NM_001686 |
| 330 | gi\|82534391 | Microtubule-associated protein tau (MAPT), transcript variant 4 | NM_016841 |
| 331 | gi\|50234883 | Zinc finger protein 358 (ZNF358) | NM_018083 |
| 332 | gi\|38455403 | Kruppel-like factor 1 (erythroid) (KLF1) | NM_006563 |
| 333 | gi\|55743084 | Asparagine-linked glycosylation 3 homolog (S. cerevisiae, alpha-1,3-mannosyltransferase) (ALG3) | NM_005787 |
| 334 | gi\|42475533 | Calsyntenin 3 (CLSTN3) | NM_014718 |
| 335 | gi\|113423957 | Huntingtin interacting protein 1 related (HIP1R) | XM_001132864 |
| 336 | gi\|4506456 | Reticulocalbin 2, EF-hand calcium binding domain (RCN2) | NM_002902 |
| 337 | gi\|22094134 | DOT1-like, histone H3 methyltransferase (S. cerevisiae) (DOT1L) | NM_032482 |
| 338 | gi\|27764862 | Solute carrier family 25 (mitochondrial carrier; adenine nucleotide translocator), member 6 (SLC25A6) | NM_001636 |
| 339 | gi\|46370065 | Exostoses (multiple) 1 (EXT1) | NM_000127 |
| 340 | gi\|4503528 | Eukaryotic translation initiation factor 4A, isoform 1 (EIF4A1) | NM_001416 |
| 341 | gi\|21359925 | High-mobility group 20A (HMG20A) | NM_018200 |
| 342 | gi\|33598925 | Scavenger receptor class B, member 2 (SCARB2) | NM_005506 |
| 343 | gi\|32880228 | Selenoprotein O (SELO) | NM_031454 |
| 344 | gi\|33695087 | Glycerol-3-phosphate dehydrogenase 1 (soluble) (GPD1) | NM_005276 |
| 345 | gi\|149087144 | Ribosomal protein, large, P0 (RPLP0), transcript variant 1 | NM_001002 |
| 346 | gi\|4758055 | CREB binding protein (Rubinstein-Taybi syndrome) (CREBBP) | NM_004380 |
| 347 | gi\|12232384 | COP9 constitutive photomorphogenic homolog subunit 7B (Arabidopsis) (COPS7B) | NM_022730 |
| 348 | gi\|90903236 | Glutathione peroxidase 4 (phospholipid hydroperoxidase) (GPX4), transcript variant 1 | NM_002085 |
| 349 | gi\|34335280 | Proteasome (prosome, macropain) 26S subunit, non-ATPase, 9 (PSMD9) | NM_002813 |
| 350 | gi\|62388867 | F-box protein 44 (FBXO44), transcript variant 4 | NM_001014765 |
| 351 | gi\|17705806 | Coenzyme Q4 homolog (S. cerevisiae) (COQ4) | NM_016035 |
| 352 | gi\|25777670 | Protein phosphatase 1, regulatory subunit 10 (PPP1R10) | NM_002714 |
| 353 | gi\|4758647 | Kinesin family member 5B (KIF5B) | NM_004521 |
| 354 | gi\|4503100 | Cysteine and glycine-rich protein 2 (CSRP2) | NM_001321 |
| 355 | gi\|54792141 | Reprimo, TP53 dependent G2 arrest mediator candidate (RPRM) | NM_019845 |
| 356 | gi\|29571103 | KiSS-1 metastasis-suppressor (KISS1) | NM_002256 |
| 357 | gi\|16306542 | Fibroblast growth factor 13 (FGF13), transcript variant 1B | NM_033642 |
| 358 | gi\|52630439 | FK506 binding protein 8, 38kDa (FKBP8) | NM_012181 |
| 359 | gi\|21735620 | Malate dehydrogenase 2, NAD (mitochondrial) (MDH2) | NM_005918 |
| 360 | gi\|13399295 | MYC-associated zinc finger protein (purine-binding transcription factor) (MAZ) | NM_002383 |
| 361 | gi\|58219047 | Hairy and enhancer of split 5 (Drosophila) (HES5) | NM_001010926 |
| 362 | gi\|67906194 | Ankyrin repeat and sterile alpha motif domain containing 6 (ANKS6) | NM_173551 |
| 363 | gi\|20127628 | Zinc finger protein 768 (ZNF768) | NM_024671 |
| 364 | gi\|4758937 | Phospholipase C, beta 2 (PLCB2) | NM_004573 |
| 365 | gi\|111496989 | Poly (ADP-ribose) polymerase family, member 1 (PARP1) | NM_001618 |
| 366 | gi\|113420584 | Similar to CXYorfl-related protein, transcript variant 1 (LOC727741) | XM_001125712 |
| 367 | gi\|32484989 | WD repeat and SOCS box-containing 2 (WSB2) | NM_018639 |
| 368 | gi\|32964831 | Hypothetical protein MGC42174 (MGC42174) | NM_152383 |
| 369 | gi\|20149616 | Neural proliferation, differentiation and control, 1 (NPDC1) | NM_015392 |
| 370 | gi\|34996486 | HesB like domain containing 1 (HBLD1) | NM_194279 |
| 371 | gi\|4758219 | Family with sequence similarity 50, member A (FAM50A) | NM_004699 |
| 372 | gi\|70609878 | Ribosomal protein S2 (RPS2) | NM_002952 |
| 373 | gi\|83267865 | Dynein, light chain, LC8-type 1 (DYNLL1), transcript variant 1 | NM_001037494 |
| 374 | gi\|113422143 | Similar to 60S ribosomal protein L21, transcript variant 2 (LOC731567) | XM_001133519 |
| 375 | gi\|113430220 | CXYorf1-related protein, transcript variant 1 (LOC376475) | XM_377073 |
| 376 | gi\|17986282 | Tubulin, alpha 3 (TUBA3) | NM_006009 |
| 377 | gi\|23238232 | High mobility group nucleosomal binding domain 4 (HMGN4) | NM_006353 |
| 378 | gi\|39725675 | CDK2-associated protein 2 (CDK2AP2) | NM_005851 |
| 379 | gi\|46389548 | Endosulfine alpha (ENSA), transcript variant 3 | NM_004436 |
| 380 | gil46389553 | Endosulfine alpha (ENSA), transcript variant 4 | NM_207044 |
| 381 | gi\|46389549 | Endosulfine alpha (ENSA), transcript variant 1 | NM_207042 |
| 382 | gi\|47078237 | G protein pathway suppressor 1 (GPS 1), transcript variant 1 | NM_212492 |
| 383 | gi\|47078280 | Family with sequence similarity 53, member B (FAM53B) | NM_014661 |
| 384 | gi\|4757715 | Sperm associated antigen 7 (SPAG7) | NM_004890 |
| 385 | gi\|50557645 | Zinc finger, FYVE domain containing 27 (ZFYVE27), transcript variant 1 | NM_001002261 |
| 386 | gi\|5174742 | Ubiquinol-cytochrome c reductase, Rieske iron-sulfur polypeptide 1 (UQCRFS 1) | NM_006003 |
| 387 | gi\|53759121 | Adenomatosis polyposis coli (APC) | NM_000038 |
| 388 | gi\|57242754 | Calsyntenin 1 (CLSTN1), transcript variant 2 | NM_014944 |
| 389 | gi\|7705480 | Ubiquitin-fold modifier conjugating enzyme 1 (UFC1) | NM_016406 |
| 390 | gi\|82659090 | Staufen, RNA binding protein, homolog 1 (Drosophila) (STAU1), transcript variant T5 | NM_001037328 |
| 391 | gi\|83641890 | Glyceraldehyde-3-phosphate dehydrogenase (GAPDH) | NM_002046 |
| 392 | gi\|57222567 | Myeloid/lymphoid or mixed-lineage leukemia (trithorax homolog, Drosophila); translocated to, 6 (MLLT6) | NM_005937 |
| 393 | gi\|14249519 | Hypothetical protein FLJ14668 (FLJ14668) | NM_032822 |
| 394 | gi\|38201669 | Inhibitor of growth family, member 4 (ING4) | NM_016162 |
| 395 | gi\|83656775 | Eukaryotic translation elongation factor 2 (EEF2) | NM_001961 |
| 396 | gi\|20127557 | Chromatin modifying protein 5 (CHMP5) | NM_016410 |
| 397 | gi\|20302149 | Lysophospholipase II (LYPLA2) | NM_007260 |
| 398 | gi\|23238257 | Carnitine palmitoyltransferase 1B (muscle) (CPT1B), nuclear gene encoding mitochondrial protein, transcript variant 4 | NM_152247 |
| 399 | gi\|33286421 | Pyruvate kinase, muscle (PKM2), transcript variant 3 | NM_182471 |
| 400 | gi\|34147626 | Zinc finger protein 447 (ZNF447) | NM_023926 |
| 401 | gi\|38176162 | Ring finger protein 130 (RNF130) | NM_018434 |
| 402 | gi\|47933378 | N-ethylmaleimide-sensitive factor attachment protein, alpha (NAPA) | NM_003827 |
| 403 | gi\|49472815 | Fascin homolog 1, actin-bundling protein (Strongylocentrotus purpuratus) (FSCN1) | NM_003088 |
| 404 | gi\|67189547 | Ribosomal protein L6 (RPL6), transcript variant 2 | NM_000970 |
| 405 | gi\|6912539 | Nucleotide binding protein 2 (MinD homolog, E. coli) (NUBP2) | NM_012225 |
| 406 | gi\|73622128 | Caspase 6, apoptosis-related cysteine peptidase (CASP6), transcript variant alpha | NM_001226 |
| 407 | gi\|83641894 | Heterogeneous nuclear ribonucleoprotein A1 (HNRPA1), transcript variant 2 | NM_031157 |
| 408 | gi\|94721349 | Islet cell autoantigen 1, 69kDa (ICA1), transcript variant 2 | NM_004968 |
| 409 | gi\|42794610 | 6-phosphogluconolactonase (PGLS), | NM_012088 |
| 410 | gi\|113420239 | Similar to block of proliferation 1 (LOC727967) | XM_001126255 |
| 411 | gi\|14149778 | Chromosome 1 open reading frame 160 (Clorf160) | NM_032125 |
| 412 | gi\|16306547 | Seryl-tRNA synthetase (SARS) | NM_006513 |
| 413 | gi\|20336240 | Proprotein convertase subtilisin/kexin type 1 inhibitor (PCSK1N) | NM_013271 |
| 414 | gi\|22027621 | TNF receptor-associated factor 4 (TRAF4), transcript variant 1 | NM_004295 |
| 415 | gi\|31543190 | Chromosome 10 open reading frame 58 (C10orf58) | NM_032333 |
| 416 | gi\|32129198 | Cytokine induced protein 29 kDa (CIP29) | NM_033082 |
| 417 | gi\|32455235 | Helicase (DNA) B (HELB) | NM_033647 |
| 418 | gi\|34452680 | Ring finger protein 10 (RNF10) | NM_014868 |
| 419 | gi\|34996518 | Galectin-3 internal gene (GALIG) | NM_194327 |
| 420 | gi\|40804743 | Sema domain, immunoglobulin domain (Ig), transmembrane domain (TM) and short cytoplasmic domain, (semaphorin) 4C (SEMA4C) | NM_017789 |
| 421 | gi\|41393582 | EGF-like-domain, multiple 7 (EGFL7), transcript variant 2 | NM_201446 |
| 422 | gi\|46430498 | V-rel reticuloendotheliosis viral oncogene homolog A, nuclear factor of kappa light polypeptide gene enhancer in B-cells 3, p65 (avian) (RELA) | NM_021975 |
| 423 | gi\|47519746 | Mitogen-activated protein kinase 11 (MAPK11) | NM_002751 |
| 424 | gi\|50233802 | NDRG family member 4 (NDRG4) | NM_020465 |
| 425 | gi\|56090145 | Hypothetical LOC339123 (LOC339123), | NM_001005920 |
| 426 | gi\|71164877 | Ribosomal protein S 12 (RPS12) | NM_001016 |
| 427 | gi\|72534683 | Phospholipase D family, member 3 (PLD3), transcript variant 1 | NM_001031696 |
| 428 | gi\|7305502 | Stomatin (EPB72)-like 2 (STOML2) | NM_013442 |
| 429 | gi\|90193629 | Septin 5 (SEPT5) | NM_002688 |
| 430 | gi\|50592995 | Tubulin, beta 3 (TUBB3) | NM_006086 |
| 431 | gi\|16554608 | Mitochondrial ribosomal protein S11 (MRPS 11), nuclear gene encoding mitochondrial protein, transcript variant 1 | NM_022839 |
| 432 | gi\|30581139 | Proteasome (prosome, macropain) activator subunit 1 (PA28 alpha) (PSME1), transcript variant 1 | NM_006263 |
| 433 | gi\|31317308 | Phosphatidylinositol-4-phosphate 5-kinase, type I, gamma (PIP5K1C) | NM_012398 |
| 434 | gi\|46048184 | Sterile alpha motif domain containing 10 (SAMD10) | NM_080621 |
| 435 | gi\|56549124 | Dynamin 2 (DNM2), transcript variant 4 | NM_001005362 |
| 436 | gi\|70166553 | GRINL1A combined protein (Gcom1), transcript variant 9 | NM_001018097 |
| 437 | gi\|40795668 | Solute carrier family 38, member 3 (SLC38A3) | NM_006841 |
| 438 | gi\|30410780 | Hypothetical protein FLJ12949 (FLJ12949), transcript variant 2 | NM_178159 |
| 439 | gi\|21361946 | Stathmin-like 4 (STMN4) | NM_030795 |
| 440 | gi\|113413590 | Similar to deoxythymidylate kinase (thymidylate kinase), transcript variant 4 (LOC727761) | XM_001126211 |
| 441 | gi\|113430465 | Similar to ataxin 7-like 3 (LOC392485) | XR_018762 |
| 442 | gi\|22219473 | Fas (TNFRSF6)-associated via death domain (FADD) | NM_003824 |
| 443 | gi\|71361681 | Nuclear mitotic apparatus protein 1 (NUMA1) | NM_006185 |
| 444 | gi\|20336312 | B-cell CLL/lymphoma 11A (zinc finger protein) (BCL11A), transcript variant 3 | NM_138559 |
| 445 | gi\|34147391 | Coiled-coil domain containing 130 (CCDC130) | NM_030818 |
| 446 | gi\|12056467 | Junction plakoglobin (JUP), transcript variant 2 | NM_021991 |
| 447 | gi\|21464122 | Methyl-CpG binding domain protein 3 (MBD3) | NM_003926 |
| 448 | gi142544170 | Excision repair cross-complementing rodent repair deficiency, complementation group 1 (includes overlapping antisense sequence) (ERCC1), transcript variant 2 | NM_001983 |
| 449 | gi\|83523747 | Hypothetical protein FLJ13305 (FLJ13305) | NM_032180 |
| 450 | gi\|46358416 | Glutamate receptor, metabotropic 3 (GRM3) | NM_000840 |
| 451 | gi\|39812062 | Mitochondrial ribosomal protein L28 (MRPL28), nuclear gene encoding mitochondrial protein | NM_006428 |
| 452 | gi\|24308369 | Nudix (nucleoside diphosphate linked moiety X)-type motif 16 (NUDT16) | NM_152395 |

## Patentansprüche

1. Verwendung der Markersequenzen zur Diagnose von Prostatakarzinom unter Ausschluss von Prostataentzündungserkrankungen oder Diabetes oder Polymorbidität, wobei mindestens eine Markersequenz einer cDNA ausgewählt aus der Gruppe SEQ 1 - 246 und / oder SEQ 247-452 oder jeweils ein dafür kodierendes Protein oder jeweils einer Teilsequenz oder Fragment davon an oder von einem zu untersuchenden Patienten bestimmt wird.

2. Verwendung der Markersequenzen zur Diagnose von Prostatakarzinom unter Ausschluss von Prostataentzündungserkrankungen oder Diabetes oder Polymorbidität nach Anspruch 1, **dadurch gekennzeichnet**, mindestens 2 bis 5 oder 10, vorzugsweise 30 bis 50 Markersequenzen oder 50 bis 100 oder mehr Markersequenzen an oder von einem zu untersuchenden Patienten bestimmt wird.

3. Verwendung der Markersequenzen zur Diagnose von Prostatakarzinom unter Ausschluss von Prostataentzündungserkrankungen oder Diabetes oder Polymorbidität nach Anspruch 1 oder 2, **dadurch gekennzeichnet, dass** die Bestimmung mittels in-vitro Diagnose erfolgt.

4. Verwendung einer Markersequenz einer cDNA jeweils ausgewählt aus der Gruppe SEQ 1 - 246 und / oder SEQ 247-452 oder jeweils ein dafür kodierendes Protein oder jeweils einer Teilsequenz oder Fragment davon als Diagnostikum.

5. Verwendung der Markersequenzen zur Diagnose von Prostatakarzinom unter Ausschluss von Prostataentzündungserkrankungen oder Diabetes oder Polymorbidität nach einem der vorhergehenden Ansprüche, **dadurch gekennzeichnet, dass** die Markersequenzen auf einem festen Träger aufgebracht werden, insbesondere einen Filter, eine Membran, ein magnetisches oder Fluorophor-markiertes Kügelchen, ein Silizium-Wafer, Glas, Metall, Kunststoff, ein Chip, ein massenspektrometrisches Target oder eine Matrix.

6. Verfahren zur Diagnose von Prostatakarzinom unter Ausschluss von Prostataentzündungserkrankungen oder Diabetes oder Polymorbidität, wobei
a.) mindestens eine Markersequenz einer cDNA ausgewählt aus der Gruppe SEQ 1 - 246 und / oder SEQ 247-452 oder jeweils ein dafür kodierendes Protein oder jeweils einer Teilsequenz oder Fragment davon auf einem festen Träger aufgebracht wird und
b.) mit Körperflüssigkeit oder Gewebeauszug eines Patienten in Kontakt gebracht wird und
c.) der Nachweis einer Wechselwirkung der Körperflüssigkeit oder Gewebeauszug mit den Markersequenzen aus a.) erfolgt.

7. Verfahren zum Stratifizieren, insbesondere zur Risikostratifizierung, oder zur Therapiesteuerung eines Patienten mit Prostatakarzinom unter Ausschluss von Prostataentzündungserkrankungen oder Diabetes oder Polymorbidität, wobei mindestens eine Markersequenz einer cDNA ausgewählt aus der Gruppe SEQ 1 - 246 und / oder SEQ 247 - 452 oder jeweils ein dafür kodierendes Protein oder jeweils einer Teilsequenz oder Fragment davon an oder von einem zu untersuchenden Patienten bestimmt wird.

8. Verfahren nach Anspruch 7, wobei das Stratifizieren oder die Therapiesteuerung Entscheidungen zur Behandlung und Therapie des Patienten, insbesondere Hospitalisierung des Patienten, Einsatz, Wirkung und / oder Dosierung eines oder mehrerer Arzneimittel, eine therapeutische Maßnahme oder die Überwachung eines Krankheitsverlaufes sowie Therapieverlauf, Ätiologie oder Klassifizierung einer Erkrankung samt Prognose umfasst.

9. Assay, Proteinbiochip bestehend aus einer Anordnung enthaltend mindestens eine Markersequenz einer cDNA jeweils ausgewählt aus der Gruppe SEQ 1 - 246 und / oder SEQ 247-452 oder jeweils ein dafür kodierendes Protein oder jeweils einer Teilsequenz oder Fragment davon, **dadurch gekennzeichnet, dass** die Markersequenzen auf einem festen Träger aufgebracht sind.

10. Verwendung eines Assays nach Anspruch 9 zum Identifizieren und Charakterisieren einer Substanz für Prostatakarzinom enthaltend Mittel zum Nachweis eines Bindungserfolges, **dadurch gekennzeichnet, dass** eine Anordnung oder Assay mit a.) mindestens einer zu untersuchenden Substanz in Kontakt gebracht wird und b.) ein Bindungserfolg nachgewiesen wird.

11. Verwendung eines Assays nach Anspruch 9 zum Screenen von Wirkstoffen für Prostatakarzinom.

12. Diagnostika zur Diagnose von Prostatakarzinom unter Ausschluss von Prostataentzündungserkrankungen oder Diabetes oder Polymorbidität, jeweils ausgewählt aus der Gruppe SEQ 1 - 246 und / oder SEQ 247 - 452 oder jeweils ein dafür kodierendes Protein oder jeweils einer Teilsequenz oder Fragment davon.

13. Target zur Behandlung und Therapie von Prostatakarzinom, jeweils ausgewählt aus der Gruppe SEQ 1 - 246 und / oder SEQ 247 - 452 oder jeweils ein dafür kodierendes Protein oder jeweils einer Teilsequenz oder Fragment davon.

14. Verwendung einer Markersequenz einer cDNA ausgewählt aus der Gruppe SEQ 1 - 246 und / oder SEQ 247 - 452 oder jeweils ein dafür kodierendes Protein oder jeweils einer Teilsequenz oder Fragment davon als Affinitätsmaterial zur Durchführung einer Apherese oder Blutwäsche für Patienten mit Prostatakarzinom.
